# EUROPEAN PATENT APPLICATION

(11) **EP 4 257 046 A1**
(43) Date of publication of application: **11.10.2023**
(21) Application number: 22166953.4
(22) Date of filing: 06.04.2022
(51) Int. Cl.: A61B 5/24, C12M 1/12, C12M 1/34, G01N 27/02, G01N 33/483, G01N 33/50, G02B 21/34

(54) **OBJECT CARRIER FOR MICROSCOPY, METHOD OF MAKING SAME AND METHOD OF MEASURING THEREWITH**

(71) Applicant: Leibniz-Institut für Neurobiologie, 39118 Magdeburg (DE); Otto-von-Guericke-Universität Magdeburg, 39106 Magdeburg (DE)
(72) Inventor: TAKAGAKI, Dr. Kentaroh, 39104 Magdeburg (DE); HERRERA-MOLINA, Dr. Rodrigo, 39104 Magdeburg (DE); ECKE, Dr. Martin, 32112 Magdeburg (DE); WILKE, Markus, 39110 Magdeburg (DE); WEBER, André, 39106 Magdeburg (DE); XIA, Zifeng, 39104 Magdeburg (DE)
(74) Representative: Günther, Constantin

(57) **Abstract**

The invention relates to an object carrier for microscopy (1) comprising an essentially plate-shaped substrate (2) which is non-conductive or at least coated with a non-conductive material wherein at least one microelectrode (4) is placed insular on the substrate (2) that can be contacted via a measuring tip (6) for the transmission of electrical signals.

The invention further relates to a method for the manufacture of an object carrier for microscopy (1) mentioned above, the method comprising
a) providing the plate-shaped substrate (2)
b) modification of the surface structure of the substrate (2) by at least one additive and/or subtractive manufacturing step by means of a focused ion beam.

## Description

### Technical Field

In a first aspect, the present invention relates to an improved object carrier for microscopy. In another aspect, the present invention relates to a method of making a previously described object carrier for microscopy. The invention furthermore relates to a method for simultaneously optical and electrical measurement and/or excitation of a sample on such an object carrier.

### Prior art

There are many samples responding to electrical stimulation or whose electrical pulses can be measured. In biological samples containing excitable cells, such as neurons, cardiac muscle cells or bone tissue, this often results in a potential difference at the cell membrane. Each cell usually exhibits its own electrical activity and/or response. The individual cells of a sample can interact with each other via electrical pulses.

Electrodes located on a carrier are already known in literature, with microelectrode arrays being an example of this. The electrodes are connected to an electronic measuring device at the edge of the carrier via conductive paths on or in the carrier. Microelectrode arrays known to date have, for example, allowed these signals to be measured between different accumulations of cells, providing, among other things, indications of the intensity of communication between neurons in response to electrical stimulation.

However, previous means have not been able to resolve communication between individual cells. Smaller electrodes can resolve single cells but not enough surrounding cells to resolve communication. Larger electrodes can observe signal from multiple cells, but due to increased noise, cannot resolve signal from each individual cell.

In addition, there are samples that similarly respond to optical stimulation or emit optical signals, such as retinal cells or samples containing pigments and/or dyes, such as fluorescent dyes. Non-biological samples or hybrid samples, such as internalized silicon nanowires, can also be subject of these studies.

Object carriers for microscopy that can be used to optically examine any sample are known. Especially high-resolution microscopy techniques include, among others, electron microscopy, scanning electron microscopy, stimulated emission depletion (STED), fluorescence lifetime microscopy, Forster resonance energy transfer microscopy, scanning tunneling microscopy, TIRF microscopy, holography, multi-photon microscropy, hyperspectral-imaging, light-sheet microscopy, and light microscopy.

A combination of the electrical and optical measurements and/or excitation is not only desirable to simplify the otherwise individually performed measurements, it also allows correlations that would not be possible in separate measurements, for example, possible changes in the sample, such as changes in cell structure in response to electrical pulses. (Chang K. Choi, Anthony E. English, Kenneth D. Kihm, Charles H. Margraves, "Simultaneous dynamic optical and electrical properties of endothelial cell attachment on indium tin oxide bioelectrodes," J. Biomed. Opt. 12(6) 064028 (1 November 2007) https://doi.org/10.1117/1.2821407)

Simultaneous electrical and optical measurement and/or excitation with a resolution of the single cell or below is not yet known. Furthermore, it would be particularly preferred to have a measuring device in which the samples can be measured over a long period of time in order to be able to observe, for example, signs of abrasion or cell growth. Further preferred would be a device that allowed this measurement in an incubator.

Thus, it is the object of the present invention to provide, manufacture and use an improved object carrier for microscopy for simultaneous optical and electrical measurement and/or excitation of a sample.

### Description of the invention

The task is solved by an object carrier for microscopy according to claim 1, which is characterized in that the microelectrode is insularly placed on the surface and the microelectrode can be contacted via at least one measuring tip for the transmission of electrical signals.

The object carrier according to the invention comprises a substrate and at least one microelectrode. The material of the substrate is electrically non-conductive or is coated with a material that is electrically non-conductive. Furthermore, the material of the substrate must be suitable for microscopy. Based on common microscopic methods, for this purpose the substrate must show at least no emission at at least one examined wavelength. Further advantageously, the substrate has no emission and reflection at at least one examined wavelength. Further advantageously, the substrate exhibits no emission and/or adsorption and/or reflection at at least one examined wavelength or range of wavelengths examined. The examined wavelength may be in the visible light range, ultraviolet radiation range, or infrared range.

The shape of the substrate is substantially plate-like, that is, it has a low height relative to its planar extent. The substrate may have indentations and/or holes. The microelectrode may be located in an indentation of the substrate.

The microelectrode is located insular on the substrate. Insular in the context of the present invention means being freestanding. In particular, the microelectrode is not connected via conductive tracks running parallel to the surface of the substrate to, for example, the edge of the object carrier. The visual field is not reduced in size by any other element, such as one or more conductor track(s) of the electrode resulting in improved optical accessibility of the sample. The microelectrode is made of electrically conductive material. The microelectrode can then be electrically contacted via the measuring tip. In this way, electrical signals at the microelectrode can be detected and/or transmitted. The invention therefore provides an object carrier for microscopy for simultaneous, non-invasive optical and electrical measurement and/or stimulation of samples, for example few to one neurons, suitable also for long-term studies and biological incubators.

According to one embodiment, the microelectrode has at least one substantially planar surface, wherein the microelectrode rests on the substrate with a substantially planar surface. The shape of the microelectrode may be planar or quasi-three-dimensional.

In another embodiment, the microelectrode is plate-shaped with at least two substantially planar parallel surfaces, of which one surface is the contact surface with the substrate. The microelectrode may be a planar, flat surface electrode. For example, the contact surface may be circular, non-cornered, triangular, rectangular, square, or polygonal.

In another embodiment, the microelectrode has at least one pillar-like structure extending away from the essentially planar surface of the at least one microelectrode and/or from the surface of the substrate. The pillar-like structure may have as a contact surface to the microelectrode only a portion of the surface of the microelectrode. The pillar-like structure may cover all or substantially all of the surface of the microelectrode not in contact with the substrate as the contact surface. The pillar-like structure may extend substantially perpendicular to the contact surface between the microelectrode and the substrate.

The pillar-like structure may be wholly or partially of conductive material. The preferred material(s) is/are biocompatible material(s). Suitable biocompatible materials are known to the skilled person. Examples of conductive biocompatible materials are gold, platinum and titanium. The pillar-like structure can serve as a contact for at least one measuring tip for detection and/or transmission of electrical signals. The pillar-like structure may be conductively connected to the microelectrode to detect and/or transmit electrical signals.

The pillar-like structure may be cuboid, cylindrical, cone-shaped or tapering. The pillar-like structure may taper towards the free end in sections or continuously. The free end of the pillar-like structure may be tapered. The free end of the pillar-like structure may be rounded. The pillar-like structure may protrude into the substrate and thus penetrate the substrate, and/or protrude from a side of the microelectrode facing away from the substrate.

A conductive pillar-like structure facilitates contact to the measuring tip.

In another embodiment, the pillar-like structure extends into or through the substrate from the microelectrode-facing surface of the substrate toward the opposite surface of the substrate, the surface of the substrate facing away from the microelectrode. The pillar-like structure penetrating the substrate may be conductively connected to the surface of the substrate facing away from the microelectrode. The substrate may include small indentations or holes for this purpose. The extension in the substrate may generate improved adhesion of the electrode to the substrate.

In this embodiment, the microelectrode may be contacted from the side facing the microelectrode via the microelectrode or an additional pillar-like structure protruding therefrom and from the substrate, as well as contacted from the side of the substrate facing away from the microelectrode via the pillar-like structure penetrating the substrate by a measurement tip for detection and/or transmission of electrical signals. Signal transmission on the substrate side facing away from the microelectrode, the side opposite the microelectrode, may further enlarge the optical accessibility.

The microelectrode may be attached solely by interactions with the surface. Suitable methods for attaching such a microelectrode are known to the skilled person. The pillar-like structure may serve as a contact on the surface of the substrate opposite the surface facing the microelectrode for the measuring tip to detect and/or transmit electrical signals.

The pillar-like structure may have an enlarged and/or widened structure and/or terminate with an enlarged and/or widened structure. The pillar-like structure may also be enlarged and/or widened on the opposite side attached to the substrate.

The pillar-like structure extending from the microelectrode may have a height of at least 2 µm high, such as 5 µm, 10 µm, 15 µm up to 20 µm, further up to 50 µm, further up to 100 µm high or more.

The contact area between the microelectrode and the substrate may be 3·10⁻⁹ m² or less, or 2·10⁻⁹ m² or less, or 1.25·10⁻⁹ m² or less, or 0.32·10⁻⁹ m² or less, or 0.08·10⁻⁹ m² or less, or 0.02·10⁻⁹ m² or less.

In another embodiment, the material of the plate-shaped substrate of the object carrier may be optically transparent. For the purposes of this application, optically transparent means that the substrate exhibits no or only low reflection and absorption as well as emission at the wavelength and/or in the wavelength range of electromagnetic radiation under investigation. The optically transparent substrate does not need to be transparent to all wavelengths. Therefore, also microscopic methods using transmitted lights in addition to those using incident light can be used.

In a further embodiment, the material of the substrate is milled glass, glass, silicon based glasses, object carriers like silicon wafer or polymer based like for example but not conclusive polydimethylsiloxane, polyester, methyl-methacrylate, dodecyl-methacrylate, octadecyl-methacrylate or polycarbonate. Also, materials used for printed circuit boards are suitable.

A conductive material may be applied between the microelectrode and the substrate, the pre-coating. The pre-coating material may comprise, for example, gold, chromium, titanium, or silver. The layer of pre-coating may be thin enough to be partially or completely transparent at one or more wavelengths. The pre-coating may be applied to the surface before the microelectrode is placed on the substrate.

According to another embodiment, the microelectrode and/or the pillar-like structure may be fully or partially coated. The material of the coating may be carbon, carbon nanotubes or a metal, such as gold, silver, aluminum, chromium, titanium, platinum.

Additional coating layer(s) of bioactive molecules like peptides or other small natural or synthetic molecules with the capacity to inhibit, modulate, regulate and/or stimulate cell function(s) can be passively via physical deposition or actively via chemical conjugation placed onto the microelectrode and/or at least one pillar-like structure coated with carbon, carbon nanotubes, or one or more metals.

The aforementioned embodiments of the pillar-like structures can be combined with each other. A microelectrode may have multiple pillar-like structures extending in different directions. The object carrier may have multiple microelectrodes that are identical or different in shape. For example, microelectrodes without pillar-like structures and microelectrodes with pillar-like structures may be present.

The experimental results so far further suggest that cells show affinity for the pillar-like structures. The effect that could be achieved is the singularization of the cells. Thus, the pillar-like structure on the microelectrode enables, or at least supports, the measurement of electrical signals from individual cells.

The microelectrode and/or the pillar-like structure may be made of biocompatible material. Biocompatible material is characterized by exhibiting minimal interactions with living cells. Examples of biocompatible material include gold, platinum, titanium, chromium.

Electrically and/or optically active cells comprise, but are not limited to, cardiac muscle cells or nerve cells and retinal cells.

Furthermore, the invention relates to a method for manufacturing an object carrier of the type described above, which is characterized in that in a first step a plate-shaped substrate is provided and in a second step the substrate is modified by at least one additive and/or subtractive manufacturing step by a focused ion beam.

A plurality of additive and/or subtractive fabrication steps can be strung together to fabricate an object carrier of the type previously described. In subtractive processes, e.g. at a current of the ion beam of approx. 1.0 nA and greater, material is removed. In additive processes, e.g. at a current of the ion beam of less than approx. 1.0 nA in combination with a suitable precursor gas, material is added. Suitable precursor gases, settings and equipment for this purpose are known to those skilled in the art. However, the specification of the current is only a guide value and a clear differentiation of subtractive and additive processes by the current is not possible.

Rather, the boundaries are blurred and depend on parameters such as residual gas pressure, area to be processed and others.

The fabrication of the microelectrode can be done directly on the substrate. Also, only a pillar-like structure can be deposited on an existing microelectrode.

The modification by the focused ion beam can be followed by a microscopic method. This allows the focused ion beam to be aligned before and/or during fabrication. Advantageously, the microscopic method can also be used to determine when the subtractive and/or additive manufacturing step can be terminated.

The provided plate-shaped substrate is e.g. made of or coated with a non-conductive material. The substrate may have indentations. The microelectrode may be located in an indentation of the substrate. The microelectrode is insularly located on the substrate. In particular, the microelectrode is not connected to the edge of the object carrier, for example, via conductive tracks running parallel to the surface of the substrate.

A microelectrode may be formed by at least one additive and/or subtractive manufacturing step on the substrate. The microelectrode may be planar or quasi-three-dimensional.

The microelectrode and one or more pillar-like structure(s) may be fabricated together in one fabrication step or a sequence of fabrication steps. In this case, several manufacturing steps can be saved.

The substrate may include indentations and/or holes by suitable methods prior to fabrication. The substrate may have indentations and/or holes by subtractive fabrication steps. Microelectrodes may be wholly or partially located in or fabricated in these indentations. Pillar-like structures may protrude into or through these indentations and/or holes.

The provided substrate may be pre-coated prior to any other described fabrication step. The pre-coating may be of a conductive material. The pre-coating may be applied by physical vapor deposition or other suitable method. The pre-coating may be optically transparent due to its material properties and/or the method of application, such as a low film thickness. The pre-coating dissipates the surface charge generated by the focused ion beam, preventing substrate from heating during the fabrication process and improving adhesion of the microelectrode.

Substrate material may be removed around the microelectrode by subtractive focused ion beam methods or other suitable methods. In one embodiment, only an optional pre-coating of the substrate is ablated around the microelectrode. With these subtractive processes, different microelectrode and pillar-like structure shapes can be created.

In one embodiment, the microelectrode and/or at least one pillar-like structure may be coated. The coating may result in an increase in the contiguous conductive surface area and therefore improve the signal-to-noise ratio as well as the adhesion of the sample. For example, the coating may be made of carbon, carbon nanotubes, or a metal, such as gold, silver, aluminum, chromium, titanium, platinum.

In one embodiment, a pillar-like structure is created by subtractive and/or additive manufacturing steps through a focused ion beam that protrudes from the microelectrode. For example, the pillar-like structure may have only a portion of the contact area of the microelectrode to the substrate as its base area. The pillar-like structure may be fabricated to extend substantially perpendicular to the contact area between the microelectrode and the substrate.

In another embodiment, the substantially planar contact area of the microelectrode to the substrate is 3·10⁻⁹ m² or less, or 2·10⁻⁹ m² or less, or 1.25·10⁻⁹ m² or less, or 0.32·10⁻⁹ m² or less, or 0.08·10⁻⁹ m² or less, or 0.02·10⁻⁹ m² or less. The smaller the contact area between the microelectrode surface and the substrate, the larger the optical measurement window.

The pillar-like structure may be made in whole or in part from the precursor gas of a conductive material. The preferred material is one or more biocompatible material(s). Suitable biocompatible materials are known to the skilled person. Examples of conductive biocompatible materials include gold, platinum and titanium. The biocompatibility prevents the material from chemical reaction with the sample.

Similarly, for non-biological samples, the material can be selected so that it does not allow reaction with the sample.

In another embodiment, the pillar-like structure is fabricated with a height of 2 µm and more, or 5 µm and more, or 10 µm and more, or 15 µm and more, up to 20 µm and more, further up to 50 µm and more, further up to 100 µm and more.

The pillar-like structure may be fabricated in a cylindrical shape, a cubic shape, or a conical shape. The pillar-like structure may be fabricated tapering toward the free end in sections or continuously. The free end of the pillar-like structure may be fabricated tapered or rounded. The rounded edges or tip may prevent the pillar-like structure to disrupt or stab the sample for non-invasive measurement.

Another aspect of the invention provides a method for bi-modal measurement and/ or stimulation of cells and/ or tissue using one of the described object carriers. The method includes optical and electrical measurement and/or stimulation of a sample. The method includes the following steps: Placing the sample on the object carrier, electrical and/or excitation of the sample via at least one measuring tip, and optical measurement and/or excitation of the sample via a microscopy technique.

That is, the sample can be either electrically and/or optically measured, electrically excited and/or optically measured, electrically measured and/or optically excited, electrically and/or optically excited, and any hybrid or juxtaposition thereof. A unique feature of this is that optical and electrical measurement and/or stimulation can be performed simultaneously or with a time delay without further modification of the device.

The electrical measurement and/or stimulation can be performed with biphasic pulses. The biphasic pulses discharge the microelectrode to avoid electrolysis and heat generation especially preserving vulnerable samples like cells (Hanson T, Fitzsimmons N, O'Doherty JE. Technology for Multielectrode MicroStimulation of Brain Tissue. In: Nicolelis MAL, editor. Methods for Neural Ensemble Recordings. 2nd edition. Boca Raton (FL): CRC Press/Taylor & Francis; 2008. Chapter 3. Available from: https://www.ncbi.nlm.nih.gov/books/NBK3896/). The electrical measurement and/or stimulation can be performed with low-impedance due to the large surface area and a lower noise level.

For the purpose of this application, a coating and a pre-coating mean the presence of the respective coating or pre-coating material on the coated or pre-coated surface. It therefore comprises surface functionalization via physisorption and chemisorption.

Besides the described fabrication process using focused ion beam, other known techniques like photoablation and photolithography might as well be suitable.

Further embodiments are:
1. An object carrier for microscopy comprising an essentially plate-shaped substrate which is non-conductive or at least coated with a non-conductive material wherein
   at least one microelectrode is placed insular on the substrate that can be contacted via a measuring tip for the transmission of electrical signals.
2. The object carrier for microscopy according to embodiment 1 wherein the at least one microelectrode has an electrically conductive base with at least one essentially planar surface contacting the substrate.
3. The object carrier for microscopy according to one of the embodiments above wherein the at least one microelectrode has at least one pillar-like structure extending away from the essentially planar surface of the at least one microelectrode and/or from the surface of the substrate.
4. The object carrier for microscopy according to embodiment 3 wherein the at least one pillar-like structure is wholly or partially of conductive material.
5. The object carrier for microscopy according to one of the embodiments 3 to 4 wherein the at least one pillar-like structure is cuboid, cylindrical, cone-shaped or tapering.
6. The object carrier for microscopy according to one of the embodiments 3 to 5 wherein the at least one microelectrode has at least one pillar-like structure sticking out from the microelectrode protruding upward, away from the substrate.
7. The object carrier for microscopy according to one of the embodiments 3 to 6 wherein the at least one microelectrode has at least one pillar-like structure which penetrates the substrate.
8. The object carrier for microscopy according to embodiment 7 wherein at least one microelectrode is conductively connected to the side of the substrate which is opposite to the side of the substrate where the at least one microelectrode is placed.
9. The object carrier for microscopy according to one of the embodiments 3 to 8 wherein the at least one microelectrode has at least one pillar-like structure which ends with an enlarged and/or widened structure.
10. The object carrier for microscopy according to one of the embodiments 6 to 8 wherein the at least one pillar-like structure is at least 2 µm high, like 5 µm, 10 µm, 15 µm up to 20 µm, further up to 50 µm, further up to 100 µm high or more.
11. The object carrier for microscopy according to one of the embodiments above wherein the area of conductive material of one microelectrode is 3.10⁻⁹ m² or less, or 2·10⁻⁹ m² or less, or 1.25·10⁻⁹ m² or less, or 0.32·10⁻⁹ m² or less, or 0.08·10⁻⁹ m² or less, or 0.02·10⁻⁹ m² or less.
12. The object carrier for microscopy according to one of the embodiments above wherein the substrate is optically transparent for electromagnetic radiation.
13. The object carrier for microscopy according to one of the embodiments above wherein the substrate material is milled glass, glass, silicon based glasses, object carriers like silicon wafer or polymer based like for example but not conclusive polydimethylsiloxane, polyester, methyl-methacrylate, dodecyl-methacrylate, octadecyl-methacrylate or polycarbonate.
14. The object carrier for microscopy according to one of the embodiments above wherein a pre-coating of a conductive material may be deposited between the at least one microelectrode and the substrate.
15. The object carrier for microscopy according to one of the embodiments above wherein the at least one microelectrode and/or the at least one pillar-like structure is coated.
16. The object carrier for microscopy according to one of the embodiments above wherein the at least one microelectrode and/or at least one pillar-like structure is coated with carbon, carbon nanotubes, or one or more metal(s).
17. The object carrier for microscopy according to embodiment 16 wherein at least one additional coating layer of bioactive molecules like peptides or other small natural or synthetic molecules with the capacity to inhibit, modulate, regulate and/or stimulate cell function(s) are passively or actively placed onto the microelectrode and/or at least one pillar-like structure coated with carbon, carbon nanotubes, or one or more metals.
18. A method for the manufacture of an object carrier for microscopy according to one of the embodiments above, the method comprising
   a) providing the plate-shaped substrate
   b) modification of the surface structure of the substrate by at least one additive and/or subtractive manufacturing step by means of a focused ion beam.
19. A method according to embodiment 18 wherein the modification is monitored by a microscopic method.
20. A method according to one of the embodiments 18 to 19 above wherein the substrate is pre-coated with a conductive material before step b).
21. A method according to one of the embodiments 18 to 20, the method comprising the coating of the microelectrode and/or at least one pillar-like structure.
22. A method according to one of the embodiments 18 to 21, the method comprising the coating of the microelectrode and/or at least one pillar-like structure with carbon, carbon nanotubes or one or more metal(s).
23. A method according to one of the embodiments 18 to 22, the method comprising the fabrication of at least one pillar-like structure extending essentially vertically to the surface of the substrate by at least one additive and/or subtractive manufacturing step by means of a focused ion beam.
24. A method according to embodiment 23, the method comprising the fabrication of the at least one pillar-like structure that it is at least 2 µm high, like 5 µm, 10 µm, 15 µm up to 20 µm, further up to 50 µm, further up to 100 µm high or more.
25. A method according to one of the embodiments 23 to 24 comprising the fabrication of at least one cuboid, cylindrical, cone-shaped or upward tapering pillar-like structure.
26. A method for bi-modal measurement and/ or stimulation of cells and/ or tissue with an object carrier, in particular an object carrier according to one of the embodiments 1 to16, the method comprising
   - Placement of the probe on the object carrier
   - Biphasic electrical measurement and/ or stimulation of cells with a contacting electrode
   - Optical measurement and/ or stimulation of the probe via microscopy technique
27. A method according to embodiment 26, the method comprising a low-impedance electrical measurement and/ or stimulation.

### Figure description

- Figure 1: shows an oblique view of an object carrier according to the invention;
- Figure 2: shows a cross-sectional view of an object carrier according to the invention with a microelectrode located in an indentation;
- Figure 3: shows a cross-sectional view of another object carrier for microscopy according to the invention with a tapering, pillar-like structure extending from a side of the microelectrode facing away from the substrate;
- Figure 4: shows a cross-sectional view of a further object carrier according to the invention with a pillar-like structure extending from a side of the microelectrode facing away from the substrate;
- Figure 5: shows a cross-sectional view of a further object carrier according to the invention with a pillar-like structure extending into the substrate;
- Figure 6: shows a cross-sectional view of a further object carrier according to the invention with a pillar-like structure extending into the substrate and terminating with an enlarged structure.

The figures are in principle comparable with each other. In the following, only the essential differences will be discussed.

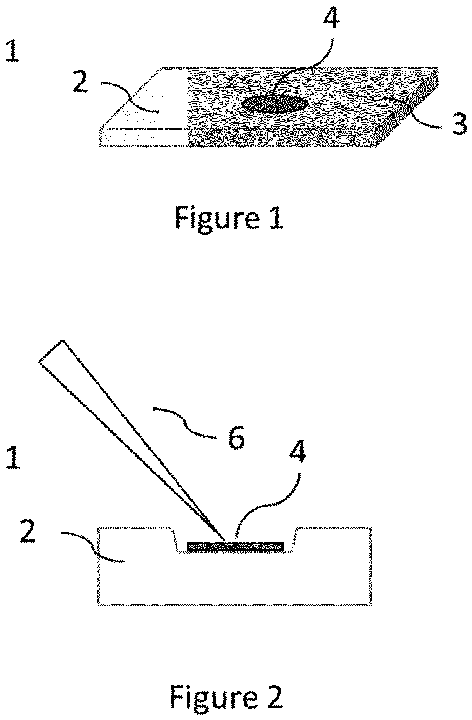

Figure 1 shows an object carrier 1 according to the invention comprising a substrate 2, a partial pre-coating 3 and an insularly placed microelectrode 4. The material of the substrate 2 is non-conductive, or at least coated with a non-conductive layer. The material of the microelectrode 4 is conductive and comprises, for example, gold, silver, chromium, titanium, platinum, or another metal. The microelectrode 4 may be located directly on the substrate 2 or on an optional pre-coating 3. The pre-coating 3 may cover portions of the substrate or the entire substrate. In particular, the microelectrode 4 may also be located in an indentation of the substrate 2. For example, the microelectrode 4 may have a circular base, but other shapes such as square or triangular or other basic shapes are also possible. The pre-coating 3 of the substrate is done before the microelectrode 4 is applied. Accordingly, the pre-coating 3 material is located between the substrate 2 and the microelectrode 4. The application of the pre-coating 3 can be done, for example, by physical vapor deposition. The pre-coating 3 material may be conductive and may comprise, for example, gold, chromium, titanium or silver. The pre-coating 3 may be applied so thinly that it is optically transparent. There may be a plurality of microelectrodes 4 on an object carrier 1, which incidentally applies to all embodiments of the invention. The microelectrode 4 may be coated.

Figure 2 shows an object carrier 1 according to the invention comprising a substrate 2 and an insularly placed microelectrode 4 as well as a measuring tip 6 contacting the microelectrode 4. The microelectrode 4 is located in an indentation of the substrate 2. The measuring tip 6 may contact the microelectrode 4 at any possible location. The measuring tip 6 can be moved laterally and vertically towards the microelectrode 4. The measuring tip 6 does not have to be in permanent contact with the microelectrode 4. The microelectrode 4 can be located directly on the substrate 2 or on an optional pre-coating 3. In particular, a microelectrode 4 according to the invention can also be located in an indentation of the substrate 2, as shown here. The microelectrode 4 has at least one substantially planar surface with which it can be attached to the substrate 2. The microelectrode 4 may be plate-shaped or have a partially curved or textured shape. For example, the microelectrode 4 may have a circular contact surface with the substrate 2, but other basic shapes such as square or triangular or other basic shapes are possible. The measuring tip 6 may contact the microelectrode 4 at any free surface. The measuring tip 6 can approach both perpendicularly and laterally to the contact surface between the substrate 2 and the microelectrode 4. The pillar-like structure 5 may be coated.

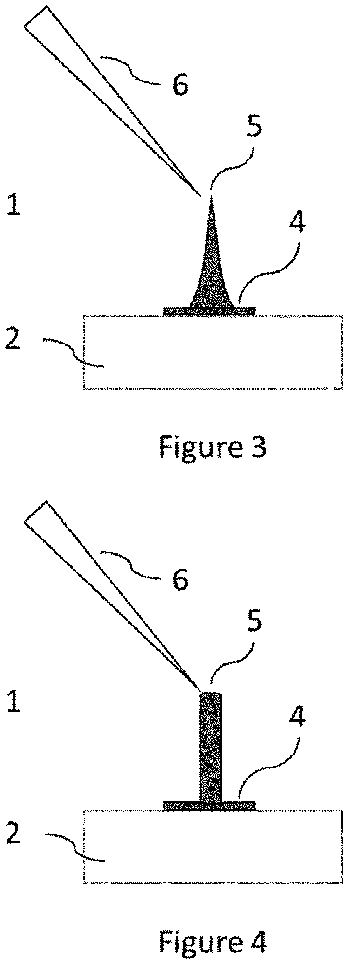

In Figure 3, a cross-section of an object carrier 1 according to the invention is shown comprising a substrate 2, an insularly placed microelectrode 4, a pillar-like structure 5 connected to the microelectrode 4, and a measuring tip 6 contacting the pillar-like structure 5. The material of the pillar-like structure 5 may be conductive and comprise, for example, gold, silver, chromium, titanium, platinum, or another metal. The pillar-like structure 5 may have a shape tapering toward the free end, as shown here in Figure 3, or it may have a cylindrical shape, but it may also be cube-shaped or cone-shaped, or otherwise taper toward the free end in a sectional or continuous manner. The pillar-like structure 5 may have a height relative to the microelectrode 4 of 2 µm or more, such as 5 µm or more, 10 µm or more, 15 µm up to 20 µm or more, further up to 50 µm or more, still further up to 100 µm or more. The pillar-like structure 5 may cover part or all of the free surface of the microelectrode 4. The measuring tip 6 may contact both the microelectrode 4 and the pillar-like structure 5 associated therewith, provided that the pillar-like structure 5 is made of electrically conductive material. The measuring tip 6 can approach both perpendicularly and laterally to the contact surface between the substrate 2 and the microelectrode 4. The measuring tip 6 may contact the pillar-like structure 5 at the tip or at any other free point. The parts of the pillar-like structure 5 extending into and enclosed by the substrate 2 are not coated.

In Figure 4, a cross-section of another object carrier 1 according to the invention is shown comprising a substrate 2, an insularly placed microelectrode 4, a pillar-like structure 5 connected to the microelectrode 4, and a measuring tip 6 contacting the pillar-like structure 5. In this embodiment, the pillar-like structure 5 has no taper towards the free end.

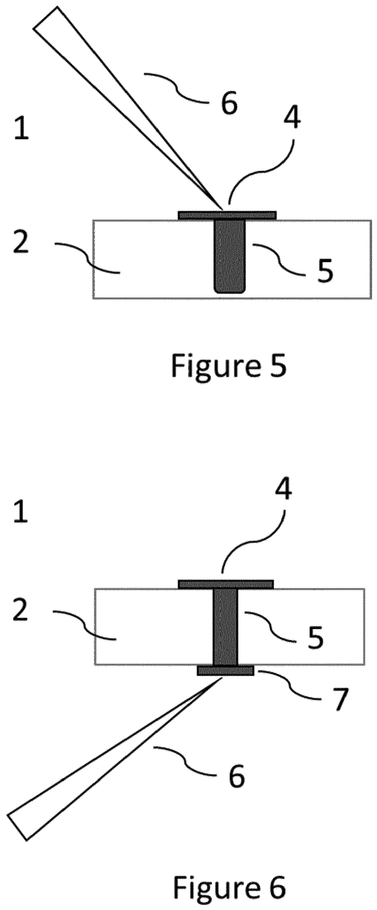

Figure 5 shows another embodiment of the object carrier 1 according to the invention comprising a substrate 2, an insularly placed microelectrode 4, a pillar-like structure 5 connected to the microelectrode 4, and a measuring tip 6 contacting the microelectrode 4. The pillar-like structure 5 can penetrate the substrate 2 by extending fully or partially into the substrate 2. For this purpose, holes may have been made in the substrate 2 before and/or during fabrication of the pillar-like structure 5. By means of such a pillar-like structure 5, a kind of through-plating of the substrate 2 can be provided.

In Figure 6, another embodiment of the object carrier 1 according to the invention is shown comprising a substrate 2, an insularly placed microelectrode 4, a pillar-like structure 5 connected to the microelectrode 4, and a measuring tip 6 contacting the microelectrode 4. The pillar-like structure 5 can penetrate the substrate 2 by extending through the substrate 2. For this purpose, holes may have been made in the substrate before and/or during fabrication of the pillar-like structure 5. The pillar-like structure 5 may terminate with an enlarged and/or widened structure 7. The measuring tip 6 may also contact the pillar-like structure on the surface of the plate-like substrate 2 facing away from the microelectrode 4. The measuring tip 6 may also contact the enlarged structure 7 of the pillar-like structure 5, provided that the pillar-like structure 5 is conductive.

## Claims

1. An object carrier for microscopy (1) comprising an essentially plate-shaped substrate (2) which is non-conductive or at least coated with a non-conductive material **wherein**
at least one microelectrode (4) is placed insular on the substrate (2) that can be contacted via a measuring tip (6) for the transmission of electrical signals.

2. The object carrier for microscopy (1) according to claim 1 wherein the at least one microelectrode (4) has an electrically conductive base with at least one essentially planar surface contacting the substrate (2)
and/or
the area of conductive material of the at least one microelectrode (4) is 3·10⁻⁹ m² or less, or 2·10⁻⁹ m² or less, or 1,25·10⁻⁹ m² or less, or 0,32·10⁻⁹ m² or less, or 0,08·10⁻⁹ m² or less, or 0,02·10⁻⁹ m² or less.

3. The object carrier for microscopy (1) according to one of the claims above wherein the at least one microelectrode (4) has at least one pillar-like structure (5) extending away from the essentially planar surface of the at least one microelectrode (4) and/or from the surface of the substrate (2).

4. The object carrier for microscopy (1) according to claim 3 wherein the at least one microelectrode (4) has at least one pillar-like structure (5) sticking out from the microelectrode (4) protruding upward, away from the substrate (2)
and/or
the at least one microelectrode (4) has at least one pillar-like structure (5) penetrating the substrate (2)
and/or
the at least one microelectrode (4) has at least one pillar-like structure (5) that is is wholly or partially of conductive material
and/or
the at least one microelectrode (4) has at least one pillar-like structure (5) that is cuboid, cylindrical, cone-shaped or tapering and/or ends with an enlarged and/or widened structure.

5. The object carrier for microscopy (1) according to one of the claims above wherein at least one microelectrode (4) is conductively connected to the side of the substrate (2) which is opposite to the side of the substrate (2) where the at least one microelectrode (4) is placed.

6. The object carrier for microscopy (1) according to one of the claims 3 to 4 wherein the at least one pillar-like structure (5) is at least 2 µm high, like 5 µm, 10 µm, 15 µm up to 20 µm, further up to 50 µm, further up to 100 µm high or more.

7. The object carrier for microscopy (1) according to one of the claims above wherein the substrate (2) is optically transparent for electromagnetic radiation and/or
the substrate (2) material is milled glass, glass, silicon based glasses, object carriers like silicon wafer or polymer based.

8. The object carrier for microscopy (1) according to one of the claims above wherein a pre-coating (3) of a conductive material may be deposited between the at least one microelectrode (4) and the substrate (2).

9. The object carrier for microscopy (1) according to one of the claims above wherein the at least one microelectrode (4) and/or the at least one pillar-like structure (5) is coated
and/or
is coated with carbon, carbon nanotubes, and/or one or more metal(s) and/or
is coated with carbon, carbon nanotubes, and/or one or more metal(s) and additional coating layer(s) of bioactive molecules like peptides or other small natural or synthetic molecules with the capacity to inhibit, modulate, regulate and/or stimulate cell function(s) passively or actively placed onto the microelectrode coated with carbon, carbon nanotubes, or one or more metals.

10. A method for the manufacture of an object carrier for microscopy (1) according to one of the claims above, **the method comprising**
a) providing the plate-shaped substrate (2)
b) modification of the surface structure of the substrate (2) by at least one additive and/or subtractive manufacturing step by means of a focused ion beam.

11. A method according to one of the claim 10 wherein the substrate (2) is pre-coated with a conductive material before step b).

12. A method according to one of the claims 10 to 11, the method comprising the fabrication of at least one pillar-like structure (5) extending essentially vertically to the surface of the substrate (2) by at least one additive and/or subtractive manufacturing step by means of a focused ion beam
and/or
the at least one pillar-like structure (5) is at least 2 µm high, like 5 µm, 10 µm, 15 µm up to 20 µm, further up to 50 µm, further up to 100 µm high or more and/or
the at least one pillar-like structure (5) is of cuboid, cylindrical, cone-shaped or upward tapering shape and/or ends with an enlarged and/or widened structure.

13. A method according to one of the claims 10 to 12, the method comprising coating of the microelectrode (4) and/or the at least one pillar-like structure (5), Wherein the coating can be carbon, carbon nanotubes and/or one or more metal(s).

14. A method for bi-modal measurement and/ or stimulation of cells and/ or tissue with an object carrier, in particular an object carrier according to one of the claims 1 to 9, **the method comprising**
• Placement of the probe on the object carrier
• Biphasic electrical measurement and/ or stimulation of cells with a contacting electrode
• Optical measurement and/ or stimulation of the probe via microscopy technique

15. A method according to claim 14, the method comprising a low-impedance electrical measurement and/or stimulation.
